# EUROPÄISCHE PATENTSCHRIFT

(11) **EP 0 673 646 B1**
(45) Veröffentlichungstag und Bekanntmachung des Hinweises auf die Patenterteilung: **02.12.1998**
(21) Anmeldenummer: 95103470.1
(22) Anmeldetag: 10.03.1995
(51) Int. Cl.: A61K 31/16

(54) **Verwendung von Deoxyspergualin zur Herstellung eines Arzneimittels zur Behandlung von entzündlich-hyperreaktiven Erkrankungen**
Use of Deoxypergualin in the manufacture of a medicament for the treatment of inflammatory-hyperresponsiveness diseases
Utilisation de déoxypergualin dans la fabrication d'un médicament pour le traitement des affections de l'hypersensibilité-inflammatoire

(30) Priorität: 22.03.1994 DE 4409804; 03.05.1994 DE 4415553
(43) Veröffentlichungstag der Anmeldung: 27.09.1995
(73) Patentinhaber: NIPPON KAYAKU CO., LTD., Chiyoda-ku, Tokyo 102 (JP); BISEIBUTSU KAGAKU KENKYUKAI, Tokyo (JP)
(72) Erfinder: Lindner, Jürgen, Dr., D-35041 Marburg (DE); Dickneite, Gerhard, Dr., D-35043 Marburg (DE); Schorlemmer, Hans Ulrich, Dr., D-35041 Marburg-Dagobertshausen (DE); Bosslet, Klaus, Dr., D-35037 Marburg (DE)
(74) Vertreter: Türk, Gille, Hrabal

(56) Entgegenhaltungen:
- EP-A- 0 184 100
- EP-A- 0 256 385
- WO-A-93/12125
- WO-A-93/16083
- JOURNAL OF HEART AND LUNG TRANSPLANTATION, Bd. 13, Nr. 1, 17.Februar 1994 Seite s41 HIRATA ET AL 'reduction of transplant arteriosclerosis with long term administration of 15-deoxyspergualin'
- Gastroenterology (1991), Bd. 101, S. 1594-1605

## Beschreibung

Die Erfindung betrifft die Verwendung von Deoxyspergualin zur Herstellung eines Arzneimittels zur Therapie und Prophylaxe von entzündlich-hyperreaktiven Erkrankungen von Mensch und Tier.

Entzündlich-hyperreaktive Erkrankungen sind dadurch definiert, daß der Körper auf unspezifische Reize mit einer überschießenden Entzündungsreaktion reagiert. Durch diese überschießende Entzündungsreaktion (Hyperreaktivität) entstehen pathologische Veränderungen, die zu einer Ausbildung und zum Chronischwerden dieser Krankheitsbilder führen. Krankheitsbilder, bei denen eine Hyperreaktivität ursächlich ist, sind entzündlich-chronische Darmerkrankungen, hyperreaktive, obstruktive Atemwegserkrankungen, entzündliche Gefäßerkrankungen, Pankreatitis, Sepsis sowie entzündlich hyperreaktive Gehirnerkrankungen wie die Alzheimersche Krankheit.

Eine entzündlich-chronische Darmerkrankung ist beispielsweise die Colitis ulcerosa. Die Pathogenese dieser Erkrankung ist nicht geklärt. Es erscheint jedoch gesichert, daß bei diesem Krankheitsgeschehen unspezifisch akut-entzündliche Reaktionen (z.B. durch Nahrungsmittelnoxen oder Infektionen) eine ursächliche Rolle spielen. Aufgrund dieser primären entzündlichen Reaktion wird die Barrierefunktion des Colonepithels gestört. Hierdurch können Noxen aus dem Darmlumen (z.B. Toxine von Anaerobiern, vor allem Clostridien) direkt auf ungeschütztes Gewebe einwirken und zu einer weiteren Verstärkung und Chronifizierung des Krankheitsgeschehens führen. Man spricht davon, daß sich eine Hyperreaktivität etabliert hat. Diese Hyperreaktivität äußert sich nicht nur in einer chronisch entzündlichen Reaktion sondern auch in schweren Tenesmen, welche für dieses Krankheitsbild typisch sind.

Die bisherige Therapie der Colitis ulcerosa besteht hauptsächlich in der Gabe von antiinflammatorisch wirkenden Substanzen wie 5-Aminosalicylsäure oder Antibiotika/Chemotherapeutika wie Metronidazol oder einigen Sulfonamidderivaten.

Unter einer hyperreaktiven obstruktiven Atemwegserkrankung versteht man beispielsweise das Bronchialasthma. Bronchialasthma ist eine Krankheit, die durch Anfälle von Atemnot (bronchiale Hyperreagibilität) charakterisiert ist, begleitet von den Zeichen einer Bronchialobstruktion, die zwischen den Anfällen spontan oder behandlungsbedingt, ganz oder teilweise reversibel ist. Unspezifische und spezifische Reize führen bei dem für Asthma typischen hyperreagiblem Bronchialbaum zur Auslösung von "Asthmaanfällen". Schleimhautödem, Bronchospasmus und Dyskrinie sind die Trias, welche die asthmatische Bronchialobstruktion kennzeichnet. Die Ursache der Hyperreaktivität des Bronchialsystems ist in einer Schädigung der Schutzmechanismen der Lunge gegenüber Noxen begründet. Flimmerepithel und Schleim sind dafür zuständig, Schadstoffe aus der Lunge zu entfernen, bevor sie zu Schädigungen des Lungengewebes und der Entstehung einer entzündlichen Reaktion führen. Bei Asthma findet man Schädigungen des Flimmerepithels und Störungen in der Zusammensetzung des Bronchialschleims. Hierdurch können Schadstoffe nicht mehr ausreichend eliminiert werden, und es entsteht eine Entzündung. Im Rahmen dieses entzündlichen Geschehens wird die Schutz- und Barrierefunktion der Lunge weiter gestört. "Harmlose" Stoffe können tiefer ins Gewebe eindringen und dort durch eine unspezifische Reizung (z.B. durch Phagozytose von Makrophagen mit anschliessender Freisetzung von Mediatoren) zu einer weiteren Steigerung und Chronifizierung der Entzündungsreaktion führen.

Wie bei entzündlichen Prozessen allgemein zu beobachten, ist auch bei Asthma die Reizschwelle (z.B. von Nervenfasern) erniedrigt. Man bezeichnet dies als Hyperreagibilität des Bronchialbaumes. Somit ist ersichtlich, daß verschiedenste Noxen, wie im folgenden aufgeführt, zu einem Asthmaanfall führen können:
- Infektionen der oberen und unteren Atemwege,
- physikalische und chemische Inhalationsnoxen (Rauch, Staub, Dämpfe, Nebel, rasche Temperaturänderungen,Gase),
- körperliche Anstrengung,
- psychische Anstrengungen (bewußt, unbewußt);
- Medikamente (z.B. Indometacin welche die Leukotriensynthese erhöhen);

Unter einer chronisch entzündlichen Gefäßerkrankung versteht man beispielsweise die Arteriosklerose. Die Arteriosklerose ist eine krankhafte Veränderung der Arterien mit Verhärtung, Verdickung, Elastizitätsverlust und Lumeneinengung. Die Pathogenese der Arteriosklerose ist abschließend nicht geklärt. Es erscheint jedoch gesichert, daß es aufgrund von verschiedensten Noxen (z.B. Hypertonie, Zigarettenrauchinhalation, Hyperlipidämie, diabetische Stoffwechsellage oder Hyperurikämie) zu einem unspezifischen entzündlichen Prozess kommt. Dieser primäre, entzündliche Prozess ist für die Entstehung einer "Hyperreaktivität" von Bestandteilen des Blutes und/oder der Blutgefäßwand verantwortlich. Bleiben die auslösenden Noxen über eine längere Zeit bestehen, so kommt es zu einer Chronifizierung und Verselbständigung der entzündlichen Reaktion, so daß dieser Prozess auch später, nach dem Wegfall der auslösenden Noxen, fortbesteht. Eine dieser auslösenden Noxen können z.B. turbulente Strömungsverhältnisse in bestimmten Gefäßabschnitten (z.B. Abzweigung der Arteria carotis communis aus dem Aortenbogen) sein. Aufgrund der durch die turbulente Strömung verursachten mechanischen Belastungen werden sowohl von der Gefäßwand als auch von den zellulären Bestandteilen des Blutes Mediatoren freigesetzt, welche über eine unspezifische Entzündungsreaktion und der damit verbundenen Hyperreaktivität zur Manifestation der Arteriosklerose führen.

Platelet Derived Growth Factor, Epidermal Growth Factor, Thromboxan und Prostaglandine sind nur einige der Mediatoren, welche zusammen mit metabolischen und zellulären Reaktionen der Gefäßwand im Rahmen dieser entzündlichen Veränderung zu beobachten sind. Aufgrund dieser Prozesse kann man ein Intimaödem, Synthesesteigerung saurer Mukopolysaccharide, Ausfällung von Lipoproteinen, Fibrinogen oder Albumin, Proliferation von Bindegewebs- und Muskelzellen mit gesteigerter Kollagen- und Elastinsynthese (Fibrose, Elastase) finden. Makroskopisch zeigt sich dieser Umbau der Gefäße im fortgeschrittenen Stadium als Verkalkung.

Die Therapie der oben beschriebenen entzündlich-hyperreaktiven Erkrankungen muß heutzutage noch immer als weitgehend unzulänglich angesehen werden. Für die Alzheimersche Krankheit, Pankreatitis und Sepsis existieren keine ausreichenden Therapiemöglichkeiten. Vor allem die starken Nebenwirkungen der eingesetzten Pharmaka stellen ein ernsthaftes Problem für den Patienten dar. Demzufolge ist die Entwicklung nebenwirkungsarmer, aber wirkungsstarker Pharmaka zur Therapie entzündlicher Erkrankungen eine medizinische Notwendigkeit.

Wir haben nun unerwarteterweise gefunden, daß 15-Deoxyspergualin [(±)-1-amino-19-guanidino-11-hydroxy-4,9,12-triazanonadecane-10,13-diontrihydrochlorid; DSG], starke Wirksamkeit in verschiedenen entzündlich-hyperreaktiven Tiermodellen zeigt. Dieser Befund ist umso unerwarteter, als die Substanz bereits seit Jahren bekannt ist und in den letzten Jahren von mehreren Arbeitsgruppen als immunsuppressive Substanz zur Behandlung von Transplantationskrisen und verschiedenen Autoimmunitätserkrankungen beschrieben wurde ( Immunomodulating Drugs, 685. 155-174; 1993).
Es kann zweckmäßig sein, das Razemat zu verwendet, doch kann auch das wirksame Stereoisomer eingesetzt werden.

Die Wirkung von DSG zur Behandlung von Mensch und Tier wird durch folgende tierexperimentelle Systeme, in denen auch in der Humanmedizin erfolgreich eingesetzte Pharmaka wirksam sind, belegt. DSG kann auch vorteilhaft in Kombination mit anderen Pharmaka eingesetzt werden.
Solche Pharmaka sind: Metronidazol, 5-Aminosalicylsäure, Salazosulfapyridin, Sulfapyridin, Antibiotika allgemein, Chemotherapeutika allgemein, Cyclosporin A, FK 506, Immunmodulatoren allgemein, Budesonid, Prednison, Prednisolon, Fluocorto-Ion, Glucocortikoide allgemein, Azathioprin, Methotrexat, Mycophenolate mofetil, Brequinar, Immunsuppressiva allgemein, Isoprenalin, Orciprenalin, β-Sympathomimetika allgemein, Theophyllin, Phosphodiesterasehemmstoffe allgemein, Ipratropium-bromid, Atropin, Parasymphatolytika allgemein, Ketotifen, Cromoglicinsäure, Mastzellstabilisatoren allgemein, Ambroxol, Carbocistein, Sekretolytika allgemein, Acetylcystein, MESNA, Mukolytika allgemein, Clemastin, Terfenadin, Antihistaminika allgemein, Reserpin, Guanfacin, Clonidin, Pindolol, β-Rezeptorenblocker allgemein, Prazosin, Minoxidil, Diazoxid, Nifedipin, Verapamil, Captopril, ACE-Hemmer allgemein, Furosemid, Diuretika allgemein, Nitroglycerin, Isosobitdinitrat, Antihypertensiva allgemein, Vasodilatatoren allgemein, Acetylsalicylsäure, Diclofenac, Phenylbutazon, Heparin, Antithrombotika allgemein, Antiinflammatorika allgemein (auch nichtsteroidale Antiphlogistika).
DSG kann oral, intravenös, subkutan, intraperitoneal, perkutan, kutan, topisch, inhalativ, intramuskulär, intrathekal, intraokulär, okulär, buccal, nasal oder rektal und in einer Dosis von 0,1 bis 100 mg/kg appliziert werden, vorzugsweise intravenös oder auch oral.
Entzündlich-hyperreaktive Erkrankungen sind beispielsweise Colitis ulcerosa, Asthma, Arteriosklerose, Pankreatitis, Sepsis oder die Alzheimersche Krankheit.

Die folgenden Beispiele erläutern die Erfindung.

### Beispiele:

Die Colitis ulcerosa wurde entsprechend der Methode von I. Okayasu et al (Gastroenterology, 1990, 98: 694 -702) mit Dextransulfat induziert. Als Verlaufsparameter der Colitis ulcerosa wurden folgende Parameter verfolgt:
- Auftreten von Blut im Stuhl; (% der positiven Tiere am letzten Versuchstag);
- Verkürzung des Dickdarmes; (Messparameter: Länge des Dickdarms in cm);
- Histologische Veränderungen; (Scoring Index: 0 = kein pathologischer Befund bis 8 = schwerste pathologische Veränderungen wie Ulzerationen, Kryptenabzeß, massives entzündliches Infiltrat)

### Beispiel 1:

### - Intraperitoneale Applikation von DSG -

### Versuchsgruppen:

I. Negativ-Kontrolle (gesunde Tiere, keine Colitis, N = 10);
II. Positiv-Kontrolle (Tiere mit Colitis, ohne Behandlung, N = 10)
III. 5 mg DSG/kg (Tiere mit Colitis, Behandlung von Tag 0 bis 9, N = 10)
IV. 7,5 mg DSG/kg (Tiere mit Colitis, Behandlung von Tag 0 bis 9; N = 10)
V. 10 mg DSG/kg (Tiere mit Colitis, Behandlung von Tag 0 bis 9, N = 10)
VI. 12,5 mg DSG/kg (Tiere mit Colitis, Behandlung von Tag 0 bis 9; N = 10)
VII. 15 mg DSG/kg (Tiere mit Colitis, Behandlung von Tag 0 bis 9; N = 10)

| **Behandlungsgruppe:** | **I:** | **II:** | **III:** | **IV:** | **V:** | **VI:** | **VII:** |
|---|---|---|---|---|---|---|---|
| Blut im Stuhl: [% positiv] | 0 % | 100 % | 80 % | 70 % | 50 % | 30 % | 10 % |
| Länge des Colons: [cm] | 6,9 ± 0,2 | 4,8 ±0,3 | 5,1 ±0,5 | 5,5 ±0,4 | 5,7 ±0,4 | 5,8 ±0,6 | 6,1 ±0,5 |
| Histologie [Scoring Index] | 0 | 4,5 ±1,1 | 4,2 ±0,7 | 3,4 ±0,6 | 3,1 ±0,8 | 2,9 ±0,6 | 2,7 ±0,8 |

### Beispiel 2:

### - Intraperitoneale Applikation von DSG -

### Versuchsgruppen:

I. Negativ-Kontrolle (gesunde Tiere, keine Colitis, N = 10);
II. Positiv-Kontrolle (Tiere mit Colitis, ohne Behandlung, N = 10);
III. Metronidazol (Tiere mit Colitis, 0,2 mg/ml, Behandlung Tag 0 bis 9, N = 10);
IV. Metronidazol (Tiere mit Colitis, 0,5 mg/ml, Behandlung Tag 0 bis 9, N = 10);
V. 7,5 mg DSG/kg (Tiere mit Colitis, Behandlung von Tag 0 bis 9; N = 10);
VI. 10 mg DSG/kg (Tiere mit Colitis, Behandlung von Tag 0 bis 9; N = 10);
VII. 7,5 mg DSG/kg in Kombination mit Metronidazol 0,2 mg/ml (Tiere mit Colitis, Behandlung von Tag 0 bis 9, N = 10);
VIII. 10 mg DSG/kg in Kombination mit Metronidazol 0,5 mg/ml (Tiere mit Colitis, Behandlung von Tag 0 bis 9, N = 10);

| **Behandlungsgruppe:** | **I:** | **II:** | **III:** | **IV:** | **V:** | **VI:** | **VII** | **VIII:** |
|---|---|---|---|---|---|---|---|---|
| Blut im Stuhl: [% positiv] | 0 % | 100 % | 80 % | 10 % | 60 % | 40 % | 10 % | 0 % |
| Länge des Colons: [cm] | 6,9 ± 0,4 | 4,9 ±0,3 | 5,7 ±0,9 | 6,0 ±0,5 | 5,7 ±0,7 | 5,9 ±1,0 | 6,2 ±0,5 | 6,9 ±0,9 |
| Histologie [Scoring Index] | 0 | 4,1 ±0,7 | 2,9 ±1,1 | 2,4 ±1,2 | 3,6 ±1,6 | 2,1 ±1,1 | 2,2 ±1,6 | 1,0 ±0,5 |

### Beispiel 3:

### - Orale Applikation von DSG -

### Versuchsgruppen

I. Negativ-Kontrolle (gesunde Tiere, keine Colitis, N = 10);
II. Positiv-Kontrolle (Tiere mit Colitis, ohne Behandlung, N = 10);
III. 10 mg DSG/kg (Tiere mit Colitis, Behandlung von Tag 0 bis 9; N = 10);
IV. 20 mg DSG/kg (Tiere mit Colitis, Behandlung von Tag 0 bis 9; N = 10);
V. 40 mg DSG/kg (Tiere mit Colitis, Behandlung von Tag 0 bis 9, N = 10);

| **Behandlungsgruppe:** | **I:** | **II:** | **III:** | **IV:** | **V:** |
|---|---|---|---|---|---|
| Blut im Stuhl: [% positiv] | 0 % | 80 % | 60 % | 40 % | 20 % |
| Länge des Colons: [cm] | 7.8 ± 0,4 | 5.5 ± 0,5 | 5,9 ± 0,7 | 6,3 ± 0,4 | 7.1 ± 0,8 |
| Histologie [Scoring Index] | 0 | 3.9 ± 0,6 | 3.1 ± 0.8 | 2,7 ± 0.7 | 2.4 ± 0.7 |

### Beispiel 4:

### Wirkung von DSG auf die IgE Synthese der Ratte (Modellerkrankung für Asthma)

In diesem Beispiel wurde untersucht, ob die für das Asthma charakteristische Erhöhung des IgE-Plasmaspiegels durch DSG zu unterdrücken ist. Dazu wurden Brown-Norway Ratten mit HgCl₂ behandelt (1 mg/kg s.c., 3x/Woche). Die IgE-Spiegel wurden im Plasma mit Hilfe der ELISA-Technik bestimmt, die Tabelle zeigt die gemessenen Extinktionen (E₄₅₀ nm), die proportional zu den IgE-Spiegeln sind.

Wie die Tabelle zeigt, ist der IgE-Spiegel in der HgCl₂-Gruppe (Gruppe 2) nach 14 Tagen gegenüber der Kontrolle (Gruppe 1) deutlich erhöht. Die Gabe von DSG (2 mg/kg, i.v., 1x täglich, von Tag 0 bis Tag 9) führte zu einer deutlichen signifikanten Unterdrückung der IgE-Spiegel (Gruppe 3). Diese Daten lassen schließen, daß DSG in der Therapie des Asthmas wirksam ist.

**Tabelle**

| Wirkung von DSG auf die HgCl₂-induzierte IgE Synthese in der Brown-Norway Ratte. | |
|---|---|
| **Gruppe** | **IgE Plasmaspiegel (E**_{**450**} **nm)** |
| 1. Kontrolle | 0.23 ± 0.06 |
| 2. HgCl₂-Behandlung | 0.59 ± 0.14 |
| 3. HgCl₂-Behandlung 1 mg/kg DSG (d0-9) | 0.29 ± 0.09 * |

| | |
|---|---|
| * p < 0.05 (t-Test) | |

### Beispiel 5:

### Wirkung von DSG auf die Arteriosklerose

Eine Arteriosklerose, gekennzeichnet durch Neointima-Bildung, wurde in der linken Arteria carotis von männlichen Ratten durch Ballonkatheterisierung (PTCA) induziert. DSG wurde in Konzentrationen von 2 und 5 mg/kg von Tag 0 bis Tag 21 Tage (tägliche i.p. Applikation) den PTCA-Ratten appliziert. Nach Beendigung des Experimentes (Tag 21) wurde die geschädigte Arteria herauspräpariert, die Bildung von Neointima wurde morphometrisch gemessen.

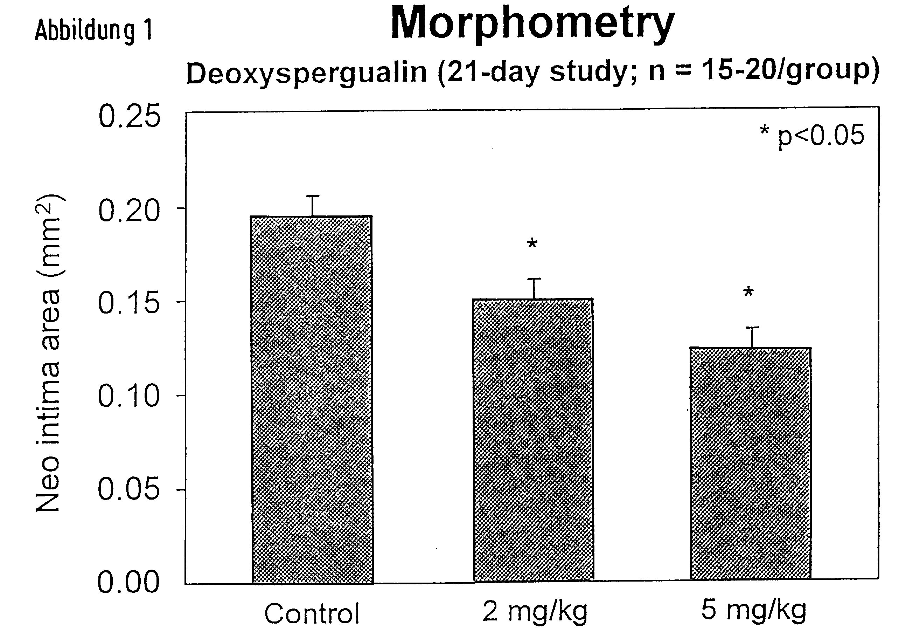

Abbildung 1 zeigt, daß die Bildung von Neointima, die auf eine arteriosklerotische Läsion der Arteria carotis beruht, durch die Gabe von DSG dosisabhängig und signifikant supprimiert wird.

## Patentansprüche

1. Verwendung von Deoxyspergualin zur Herstellung eines Arzneimittels zur Therapie von und Prophylaxe gegen entzündlich-hyperreaktive Erkrankungen.

2. Verwendung nach Anspruch 1, dadurch gekennzeichnet, daß das wirksame Stereoisomer verwendet wird.

3. Verwendung nach Anspruch 1, dadurch gekennzeichnet, daß die Erkrankung eine chronisch entzündliche Darmerkrankung, eine hyperreaktive obstruktive Atemwegserkrankung, eine chronisch entzündliche Gefäßerkrankung oder eine chronisch entzündliche Erkrankung des Gehirns ist.

4. Verwendung nach Anspruch 1, dadurch gekennzeichnet, daß die Erkrankung Colitis ulcerosa, Asthma, durch unspezifische Entzündungsreaktion und damit verbundene Hyperreaktivität ausgelöste Arteriosklerose, Pankreatitis, Sepsis oder die Alzheimersche Krankheit ist.

5. Verwendung nach Anspruch 1, dadurch gekennzeichnet, daß eine Kombination mit einem weiteren Wirkstoff hergestellt wird.

6. Verwendung nach Anspruch 1, dadurch gekennzeichnet, daß eine Kombination mit Metronidazol hergestellt wird.

7. Verwendung nach Anspruch 1, dadurch gekennzeichnet, daß ein Arzneimittel enthaltend Deoxyspergualin für eine Dosierung von 0,1 bis 100 mg/kg hergestellt wird.

8. Verwendung nach Anspruch 1, dadurch gekennzeichnet, daß ein Arzneimittel zur peroralen, intravenösen, subkutanen, intrakutanen, intraperitonealen, intrathekalen, intraokulären, okulären, buccalen, nasalen, perkutanen, kutanen, topischen, inhalativen, intramuskulären oder rektalen Applikation hergestellt wird.

9. Verwendung nach Anspruch 1, dadurch gekennzeichnet, daß ein Arzneimittel zur intravenösen oder oralen Applikation hergestellt wird.

## Claims

1. The use of deoxyspergualin for preparing a pharmaceutical for the therapy of, and prophylaxis against, hyperreactive inflammatory diseases.

2. The use as claimed in claim 1, wherein the active stereoisomer is used.

3. The use as claimed in claim 1, wherein the disease is a chronic inflammatory intestinal disease, a hyperreactive, obstructive respiratory tract disease, a chronic inflammatory vascular disease or a chronic inflammatory disease of the brain.

4. The use as claimed in claim 1, wherein the disease is ulcerative colitis, asthma, arteriosclerosis which is triggered by nonspecific inflammatory reaction and hyperreactivity associated therewith, pancreatitis, sepsis or Alzheimer's disease.

5. The use as claimed in claim 1, wherein a combination with an additional active compound is prepared.

6. The use as claimed in claim 1, wherein a combination with metronidazole is prepared.

7. The use as claimed in claim 1, wherein a pharmaceutical containing deoxyspergualin is prepared for a dosage of from 0.1 to 100 mg/kg.

8. The use as claimed in claim 1, wherein a pharmaceutical is prepared for peroral, intravenous, subcutaneous, intracutaneous, intraperitoneal, intrathecal, intraocular, ocular, buccal, nasal, percutaneous, cutaneous, topical, inhalative, intramuscular or rectal administration.

9. The use as claimed in claim 1, wherein a pharmaceutical is prepared for intravenous or oral administration.

## Revendications

1. Utilisation de la désoxyspergualine pour la fabrication d'un médicament destiné au traitement et à la prophylaxie de maladies d'hypersensibilité-inflammatoires.

2. Utilisation selon la revendication 1, caractérisée en ce que l'on utilise le stéréoisomère actif.

3. Utilisation selon la revendication 1, caractérisé en ce que la maladie est une maladie intestinale inflammatoire chronique, une maladie hyper-réactive obstructive des voies respiratoires, une maladie vasculaire inflammatoire chronique ou une maladie inflammatoire chronique du cerveau.

4. Utilisation selon la revendication 1, caractérisée en ce que les maladies sont la colite ulcéreuse, l'asthme, l'artériosclérose déclenchée par une réaction inflammatoire non spécifique et l'hypersensibilité qui y est liée, la pancréatite, la septicémie ou la maladie d'Alzheimer.

5. Utilisation selon la revendication 1, caractérisée en ce que l'on prépare une association avec une autre substance active.

6. Utilisation selon la revendication 1, caractérisée en ce que l'on prépare une association avec du métronidazole.

7. Utilisation selon la revendication 1, caractérisée en ce que l'on fabrique un médicament contenant de la désoxyspergualine à une dose de 0,1 à 100 mg/kg.

8. Utilisation selon la revendication 1, caractérisée en ce que l'on fabrique un médicament destiné à l'administration orale, intraveineuse, sous-cutanée, intracutanée, intrapéritonéale, intrathécale, intraoculaire, oculaire, gingivo-jugale, nasale, percutanée, dermique, locale, par inhalation, intramusculaire ou rectale.

9. Utilisation selon la revendication 1, caractérisée en ce que l'on fabrique un médicament destiné à l'administration orale ou intraveineuse.
